(19) Europäisches Patentamt
European Patent Office
Office européen des brevets

(11) **EP 1 438 394 B1**

(12) **FASCICULE DE BREVET EUROPEEN**

(45) Date de publication et mention
de la délivrance du brevet:
**24.01.2007 Bulletin 2007/04**

(51) Int Cl.:
***C12N 5/06*** *(2006.01)*     ***C12N 5/10*** *(2006.01)*
***C12Q 1/02*** *(2006.01)*

(21) Numéro de dépôt: **02793178.1**

(22) Date de dépôt: **22.10.2002**

(86) Numéro de dépôt international:
**PCT/FR2002/003616**

(87) Numéro de publication internationale:
**WO 2003/035853 (01.05.2003 Gazette 2003/18)**

(54) **CULTURES DE MASTOCYTES DE PORC ET LEURS UTILISATIONS**

MASTZELLKULTUREN AUS DEM SCHWEIN UND DEREN VERWENDUNGEN

PIG MAST CELL CULTURES AND USES THEREOF

(84) Etats contractants désignés:
**AT BE BG CH CY CZ DE DK EE ES FI FR GB GR IE IT LI LU MC NL PT SE SK TR**

(30) Priorité: **22.10.2001 FR 0113608**

(43) Date de publication de la demande:
**21.07.2004 Bulletin 2004/30**

(73) Titulaires:
• **INSTITUT NATIONAL DE LA RECHERCHE AGRONOMIQUE (INRA)**
**75007 Paris (FR)**
• **Ecole Nationale Vétérinaire de Maisons ALFORT ENVA**
**94704 Maisons Alfort (FR)**

(72) Inventeurs:
• **FEMENIA, Françoise**
**F-94100 Saint-Maur-Des-Fosses (FR)**
• **DELOUIS, Claude**
**F-78350 Jouy-en-Josas (FR)**
• **PARODI, André, Laurent**
**F-94210 Saint-Maur-la-Varenne (FR)**
• **LILIN, Thomas,**
**Ecole Nationale Vétérinaire**
**F-94704 Maisons Alfort (FR)**
• **AROCK, Michel**
**F-75014 Paris (FR)**

(74) Mandataire: **Vialle-Presles, Marie José et al Cabinet ORES,**
**36, rue de St Pétersbourg**
**75008 Paris (FR)**

(56) Documents cités:
• **EMERY DAVID W ET AL: "Enhancement of swine progenitor chimerism in mixed swine/human bone marrow cultures with swine cytokines." EXPERIMENTAL HEMATOLOGY (CHARLOTTESVILLE), vol. 27, no. 8, août 1999 (1999-08), pages 1330-1337, XP002228914 ISSN: 0301-472X**
• **ISHIZAKA TERUKO ET AL: "Development of human mast cells from their progenitors." CURRENT OPINION IN IMMUNOLOGY, vol. 5, no. 6, 1993, pages 937-943, XP008005659 ISSN: 0952-7915 cité dans la demande**
• **EMERY DAVID W ET AL: "Culture and characterization of hematopoietic progenitor cells from miniature swine." EXPERIMENTAL HEMATOLOGY (CHARLOTTESVILLE), vol. 24, no. 8, 1996, pages 927-935, XP008006037 ISSN: 0301-472X**
• **RAZIN E ET AL: "CULTURE FROM MOUSE BONE MARROW OF A SUBCLASS OF MAST CELLS POSSESSING A DISTINCT CHONDROITIN SULFATE PROTEO GLYCAN WITH GLYCOSAMINO GLYCANS RICH IN N ACETYL GALACTOSAMINE 4 6 DI SULFATE" JOURNAL OF BIOLOGICAL CHEMISTRY, vol. 257, no. 12, 1982, pages 7229-7236, XP008005645 ISSN: 0021-9258 cité dans la demande**
• **ZHANG Z ET AL: "PORCINE STEM CELL FACTOR/C-KIT LIGANDS: ITS MOLECULAR CLONING AND LOCALIZATION WITHIN THE UTERUS" BIOLOGY OF REPRODUCTION, SOCIETY FOR THE STUDY OF REPRODUCTION, CHAMPAIGN, IL, US, vol. 50, no. 1, 1994, pages 95-102, XP000609781 ISSN: 0006-3363**

**Description**

L'invention est relative à des cultures de mastocytes de porc et à leurs utilisations.

Les mastocytes sont des cellules du système immunitaire, qui interviennent dans la réponse inflammatoire, notamment dans les phénomènes d'allergie et d'hypersensibilité. Elles sont localisées dans le tissu conjonctif, notamment au niveau de la peau, des tractus digestif et respiratoire, et dans les muqueuses intestinales et respiratoires. Il existe également un petit nombre de mastocytes dans la moelle osseuse et dans les organes lymphoïdes.

Les mastocytes matures, quelle que soit leur localisation, ont en commun certaines caractéristiques telles que la présence de nombreuses granulations intra cytoplasmiques métachromatiques. Il s'agit de cellules arrondies d'un diamètre de 13 à 22 $\mu$m ; elles possèdent un noyau arrondi, unique, central ou le plus souvent excentré. Le cytoplasme est entièrement rempli de granulations, l'abondance des granulations recouvrant parfois une partie du noyau.

Ces granulations renferment différentes substances chimiques synthétisées par les mastocytes, parmi lesquelles on citera notamment l'histamine, la sérotonine, des protéoglycannes tels que l'héparine ou le chondroïtine sulfate, des enzymes, notamment des protéases, des cytokines telles que le TNF-alpha, et des facteurs "chimioattractants" des éosinophiles et des neutrophiles (ABRAHAM et MALAVIYA, Infection and Immunity, 65, 3501, 1997). Ces substances pro-inflammatoires sont libérées brutalement (phénomène dénommé « dégranulation ») lors de l'activation mastocytaire. Dans un deuxième temps se met en place une réponse secondaire, liée à la synthèse *de novo* de médiateurs tels que les leucotriènes, prostaglandines, PAF *(platelet activating factor),* mais aussi d'interleukines (IL4, IL5, IL6, IL10, IL12, IL13), de cytokines (TGF-beta, IFN-gamma, GM-CSF) et de chimiokines (MCP-1, IL8, RANTES) (BEFUS, Reg. Immunol., 2, 176, 1989 ; MOQBEL *et al.,* Immunology, 60, 425, 1987). L'ensemble de ces facteurs participe activement à l'enclenchement d'un processus inflammatoire et à la mise en place d'une réponse immunitaire spécifique dépendante des lymphocytes T.

Les mastocytes constituent en fait une population cellulaire très hétérogène.

Deux sous-populations mastocytaires distinctes qui présentent des caractéristiques biochimiques et fonctionnelles très différentes ont été caractérisées : les mastocytes muqueux et les mastocytes séreux. Ces deux sous-populations peuvent être différenciées par les substances actives élaborées et stockées dans les granules. Ainsi, chez la souris les mastocytes muqueux produisent principalement de l'histamine, de la chymase et du chondroïtine sulfate A et E, et les mastocytes séreux produisent principalement de l'histamine, de la sérotonine, de la chymase, de la tryptase et de l'héparine.

Chez l'homme les mastocytes muqueux également dénommés $MC_T$ (tryptase +) produisent principalement de l'histamine, de la tryptase, de l'héparine, et du chondroïtine sulfate A et E, et les mastocytes séreux également dénommés $MC_{TC}$ (tryptase + et chymase +) produisent en outre de la chymase.

Les mastocytes sont dérivés de précurseurs hématopoïétiques (GALLI, Lab. Invest. 62, 5-33, 1990). Des populations de mastocytes ont été obtenues à partir de cultures de moelle osseuse de souris (RAZIN *et al.,* J. Biol. Chem., 257, 7229-7236, 1982; DAYTON *et al.,* Proc. Natl. Acad. Sci. USA, 85, 569-572, 1988) en présence de milieux conditionnés ou en présence d'IL3. A partir d'une suspension de cellules hématopoïétiques de souris, on obtient une population pure de mastocytes en 3 semaines.

L'obtention de cultures *in vitro* de mastocytes humains s'est avérée plus difficile. Des cultures de cellules de moelle osseuse ou de cordon ombilical en présence d'IL3 ont conduit à l'apparition de granulocytes basophiles (TADOKORO *et al.,* J. Exp. Med., 158, 857-871, 1983). La co-culture de cellules de cordon ombilical humain avec des fibroblastes 3T3 de souris a toutefois permis d'obtenir des mastocytes matures après-quatre semaines de culture (ISHIZAKA *et al.,* Current Opinion in Immunology, 5, 937-943, 1993 ; FURITSU *et al.*, Proc. Natl. Acad. Sci. USA, 86, 10039-10043, 1989). Il a ensuite été montré (ISHIZAKA *et al.*, publication précitée) que la différenciation et la prolifération des mastocytes humains *in vitro* dépend du ligand c-kit (également dénommé SCF pour : « Stem Cells Factor ») produit par les cellules 3T3.

Les cultures de mastocytes constituent un outil utile pour l'étude des mécanismes impliqués dans différents phénomènes inflammatoires et/ou immunitaires, par exemple dans le cadre de la réponse allergique, ou de la réponse immunitaire à l'agression par divers pathogènes, notamment des parasites. Cependant on n'a jusqu'à présent réussi à différencier et maintenir *in vitro* des cultures de mastocytes que dans le cas des cellules humaines ou de rongeurs ; en outre on ne dispose actuellement que d'un petit nombre de lignées de mastocytes.

La présente invention a pour but de fournir des cultures de mastocytes provenant du porc, et des lignées de mastocytes issues de ces cultures. En effet, outre son intérêt économique, le porc constitue un modèle de plus en plus utilisé dans le cadre d'études physiologiques et cliniques. D'autre part, le porc héberge des parasites transmissibles à l'homme (Trichinella, cysticerques, toxoplasme), et l'étude du rôle joué par les mastocytes dans les mécanismes de défense vis-à-vis de ceux-ci, présente un grand intérêt du point de vue de la santé animale et de la santé publique (prophylaxie médicale).

Des études précédentes ont montré que l'établissement de lignées de mastocytes humains et de rongeurs uniquement isolées à partir de tumeurs (mastocytomes) nécessitait la présence d'une seule cytokine spécifique dans le milieu de culture.

La présence de mastocytomes de porc est une éventualité extrêmement rare, ce qui a interdit à ce jour, l'obtention de lignées de mastocytes de cette espèce.

Les Inventeurs sont maintenant parvenus à mettre au point une méthode de culture, en présence de 2 cytokines spécifiques, permettant l'obtention, à partir de précurseurs hématopoïétiques du foie ou de la moelle osseuse, d'une population pure de mastocytes porcins, et son maintien en culture pendant une très longue durée.

Ils sont en outre parvenus à obtenir, à partir de cette culture, des cellules immortalisées, conservant les caractéristiques des mastocytes d'origine.

La présente invention a en conséquence pour objet un procédé d'obtention d'une culture de mastocytes porcins caractérisé en ce qu'il comprend la mise en culture de cellules souches obtenues à partir du foie ou de la moelle osseuse d'un foetus de porc, dans un milieu comprenant au moins 0,2 ng/ml, avantageusement de 0,5 à 10 ng/ml, de préférence de 1 à 5 ng/ml, et de manière tout à fait préférée environ 2 ng/ml d'IL-3 porcin, et au moins 8 ng/ml, avantageusement de 20 à 200 ng/ml, de préférence de 40 à 120 ng/ml, et de manière tout à fait préférée environ 80 ng/ml de SCF porcin.

Selon un mode de mise en oeuvre préféré de la présente invention, les cellules sont cultivées dans ce milieu pendant au moins 60 à 70 jours pour obtenir 100% de mastocytes.

Selon un autre mode de mise en oeuvre préféré de la présente invention, ledit procédé comprend en outre la transformation de ladite culture par un oncogène immortalisant.

La présente invention a également pour objet toute culture de mastocytes porcins susceptible d'être obtenue par le procédé conforme à l'invention.

Au moins 90%, de préférence au moins 95%, et de manière tout à fait préférée au moins 99% des cellules de cette culture présentent les caractéristiques suivantes :

- elles renferment des granulations contenant de l'histamine et de l'héparine;
- elles expriment le récepteur c-kit et le récepteur des IgE.

La présente invention englobe notamment toute sous-population, lignée, ou clone de mastocytes pouvant être obtenu à partir des cultures de mastocytes porcins conformes à l'invention.

Le terme « culture » désigne ici, de manière générale, une cellule ou un ensemble de cellules cultivées *in vitro*. Une culture développée directement à partir d'un prélèvement cellulaire ou tissulaire effectué sur un animal est dénommée « culture primaire ». Le terme « lignée » est employé à partir du moment où au moins un passage, et généralement plusieurs passages consécutifs en sous-culture ont été effectués avec succès, et désigne toute culture qui en est issue. Le terme « clone » désigne un ensemble de cellules issues d'une seule cellule d'une culture primaire ou d'une lignée (SCHAEFFER, In Vitro Cellular and Developmental Biology, 26, 91-101, 1990).

La présente invention a ainsi plus particulièrement pour objet :

- une lignée de mastocytes issus de cellules souches du foie foetal de porc, déposée auprès de la CNCM (Collection Nationale de Cultures de Microorganismes, Institut Pasteur, 26 rue du Docteur Roux, 75724 PARIS CEDEX 15, France) le 17 octobre 2001, sous le numéro I-2735.
- une lignée de mastocytes issus de cellules souches du foie foetal de porc et transfectés par l'oncogène T du virus SV40, déposée auprès de la CNCM le 17 octobre 2001, sous le numéro I-2736.
- une lignée de mastocytes issus de cellules souches de la moelle osseuse d'un foetus de porc et transfectés par l'oncogène T du virus SV40, déposée auprès de la CNCM le 17 octobre 2001, sous le numéro I-2734.

Toutes ces cellules peuvent être cultivées dans les conditions définies ci-dessus.

Le maintien en culture ne nécessite toutefois pas la présence d' IL-3, et le SCF peut être utilisé à des doses plus faibles que celles utilisées pour l'obtention des cultures. Ainsi on peut utiliser le SCF à des concentrations à partir de 2 ng/ml, de préférence à des concentrations de 5 à 100 ng/ml, avantageusement de 8 à 80 ng/ml. Ces cellules peuvent également être maintenues en culture en l'absence de SCF et d'IL-3 pendant environ 2 mois.

Les mastocytes conformes à l'invention peuvent notamment être utilisés comme modèles pour étudier l'activation mastocytaire en réponse à un allergène, ou à un parasite. Ils présentent un intérêt tout particulier en tant que modèle *in vitro* pour l'étude des mécanismes immunitaires qui sont impliqués dans l'acquisition d'une immunité protectrice vis-à-vis de nématodes parasites. A titre d'exemple, on citera l'étude des mécanismes impliqués dans la mise en place d'une immunité locale après l'infestation des cellules épithéliales intestinales par *Trichinella*.

Les mastocytes conformes à l'invention peuvent également être utilisées pour identifier les antigènes intervenant dans l'activation des mastocytes non seulement chez le porc, mais également chez d'autres animaux, y compris l'homme, en particulier dans le cadre du criblage d'antigènes potentiellement utilisables pour l'obtention de vaccins ou de réactifs de diagnostic. On peut notamment tester ainsi des antigènes recombinants ou synthétiques, sur la base de leur capacité à activer *in vitro* ces mastocytes.

Dans le cas de *Trichinella* par exemple, ces antigènes peuvent avoir des applications pour le diagnostic des trichinelloses animales (diagnostic précoce de la trichinellose des suidés), ou pour le développement de vaccins (par exemple vaccination par voie mucosale).

La présente invention sera mieux comprise à l'aide du complément de description qui va suivre, qui se réfère à des exemples non-limitatifs décrivant l'obtention de cultures et de lignées de mastocytes porcins conformes à l'invention.

**EXEMPLE 1 : ISOLEMENT DE POPULATIONS PURES DE MASTOCYTES A PARTIR DE MOELLE OU DE FOIE DE FOETUS DE PORC**

**Prélèvement et mise en culture des cellules**

Les prélèvements ont été réalisés à partir de 7 foetus d'une truie Meishan au 77ème jour de gestation ; 7 foies et 8 fémurs ont été prélevés.

Récolte des cellules du foie

Les foies ont été broyés et les cellules individualisées avec 20 ml de trypsine 0,5% EDTA 5 mM, dans un bain marie à 37°C pendant 15 mn. Après incubation, une filtration sur des gazes stériles a été réalisée en effectuant plusieurs rinçages avec du milieu DMEM (GIBCO), supplémenté en SVF à 15%, Pénicilline/Streptomycine 100 U/ml, Glutamine 2 mM (milieu DMEM complet).

Après une centrifugation de 5 mn à 1000 g, un gradient de ficoll a été utilisé afin d'éliminer les hématies ; le culot est repris dans 25 ml de milieu DMEM pour 17 ml de ficoll. Une centrifugation a été effectuée à 1500 g pendant 25 mn, l'anneau de cellules blanches est ensuite prélevé et rincé deux fois en milieu DMEM complet.

Le culot a été ensuite récupéré dans 10 ml de milieu MEM $\alpha$ (GIBCO) et une numération cellulaire a été réalisée (soit $2.10^6$ cellules/ml).

Les cellules ont été ensemencées, à raison de $4.10^6$ cellules/ml, dans une flasque avec 5 ml de milieu MEM $\alpha$ supplémenté en SVF (sérum de veau foetal) à 15%, Pénicilline/Streptomycine 100 U/ml, Glutamine 2 mM, PGE2 (prostaglandine E, Sigma) à 1 $\mu$M final (milieu MEM $\alpha$ complet), en présence d'IL3 (2 ng/ml) et de SCF (80 ng/ml) porcins recombinants (BIOTRANSPLANT).

Récolte des cellules de la moelle osseuse

Les fémurs de foetus ont été coupés à l'aide de ciseaux à chaque extrémité et la moelle osseuse a été récoltée par perfusion à l'aide d'une seringue contenant du PBS.

Une fois rincées en PBS, les cellules ont été ensemencées, à raison de $2,4.10^6$ cellules/ml, dans une flasque avec 20 ml de milieu MEM $\alpha$ complet en présence d'IL3 porcin (2 ng/ml) et de SCF porcin (80 ng/ml).

Culture des cellules

Les flasques sont placées à l'étuve à 37°C en présence de $CO_2$ (à 5%).

Les cellules sont réensemencées toutes les semaines à raison de $10^6$ cellules/ml, dans du milieu MEM $\alpha$ complet en présence d'IL3 porcin (2 ng/ml) et de SCF porcin (80 ng/ml).

Des échantillons sont prélevés à intervalles réguliers pour effectuer une numération cellulaire. Après cytocentrifugation et coloration au bleu de toluidine, le pourcentage de mastocytes est obtenu par comptage au microscope.

Les résultats sont rapportés dans le tableau I ci-après.

TABLEAU I

| Age de la Culture en jours | Cellules de moelle osseuse $10^6$/ ml | Mastocytes % | Cellules de foie $10^6$/ml | % Mastocytes |
|---|---|---|---|---|
| 5 | 0,7 | 0 | 3 | 0 |
| 12 | 0,5 | rares | 0,6 | 70 |
| 19 | 0,9 | 5 | 0,4 | 80 |
| 26 | 0,4 | 30 | 0,4 | 80 |
| 33 | 0,5 | 70 | 0,3 | 80 |
| 40 | 0,7 | 80 | 0,3 | 80 |
| 47 | 0,3 | 80 | 0,4 | 80 |

(suite)

| Age de la Culture en jours | Cellules de moelle osseuse $10^6$/ ml | Mastocytes % | Cellules de foie $10^6$/ml | % Mastocytes |
|---|---|---|---|---|
| 54 | 0,6 | 90 | 0,4 | 90 |
| 60 | 1,3 | 100 | 0,6 | 90 |
| 67 | 1,7 | 100 | 1,1 | 100 |
| 74 | 1 | 100 | 0,8 | 100 |
| 82 | 1,8 | 100 | 1,1 | 100 |
| 89 | 1 | 100 | 0,8 | 100 |
| 90 | 1 | 100 | 0,7 | 100 |
| 97 | - | - | 1,3 | 100 |
| 104 | - | - | 0,9 | 100 |

La cinétique des cultures, quelle que soit leur provenance tissulaire, révèle qu'après une période de 60 jours (moelle osseuse) et de 67 jours (foie foetal), la population cellulaire subit un accroissement sensible, notamment pour les cellules d'origine médullaire. Cet accroissement correspond au passage d'une culture mixte à une population homogène 100% de mastocytes.

Ces cellules demeurent capables de se multiplier après congélation/décongélation.

A ce jour, après 714 jours de culture, les mastocytes sont viables et continuent de se multiplier.

### Caractérisation des cellules

Les mastocytes sont mis en évidence par coloration au bleu de toluidine et coloration de May Grünwald et Giemsa. L'héparine et l'histamine des granulations sont révélées par coloration au sulfate de berbérine et au bleu alcian-safranine.

Les mastocytes en culture apparaissent arrondis avec un rapport nucléo-cytoplasmique élevé. Le noyau est arrondi, le plus souvent central ou excentré non segmenté ; dans le cytoplasme les granulations sont nombreuses, de couleur violet foncé à rouge ; le nombre et la taille des granulations varient : elles sont petites et peu nombreuses dans les cellules considérées comme immatures ; elles sont nombreuses et volumineuses dans les cellules considérées comme matures.

Après coloration au sulfate de berbérine, on observe au microscope à fluorescence des granulations colorées dans le cytoplasme des mastocytes, correspondant aux granules contenant de l'héparine. La quantité d'héparine apparaît variable d'une cellule à l'autre ; cette variation peut être en relation avec le stade de maturation des cellules.

Après coloration au bleu alcian-safranine, les grains d'héparine sont colorés en rouge par la safranine et les grains d'histamine en bleu par le bleu alcian. On observe dans certaines cellules un fort marquage de l'héparine, comme dans le cas de la coloration par la berbérine.

La caractérisation morphologique a été complétée par une observation en microscopie électronique.

Les mastocytes de porc observés sont des cellules de grande taille (16 $\mu$m). Ils ont un gros noyau clair, souvent excentré, à contour régulier, avec une chromatine légèrement marginée. Dans le noyau, on observe un à deux nucléoles volumineux en position centrale. Le cytoplasme est assez abondant, il contient des ribosomes, de nombreuses mitochondries volumineuses, et de nombreuses granulations.

Les granulations apparaissent entourées d'une membrane simple, contenant un matériel plus ou moins dense aux électrons, d'aspect irrégulier avec des particules petites ou moyennes, parfois des structures en forme de bâtonnets plus ou moins réguliers. Ces granulations sont du même type que celles décrites dans des mastocytes humains en culture (EGUCHI, Electron. Microsc. Rev., 4(2), 293-318, 1991).

Mise en évidence du récepteur c-kit et du récepteur des IgE

La présence de ces récepteurs a été mise en évidence par immunohistochimie.

*Récepteur c-kit*

Ce récepteur a été détecté en utilisant l'anticorps polyclonal C19 de lapin anti c-Kit humain (TEBU), qui reconnaît également le récepteur c-kit porcin.

Des marquages indirects à différents temps de culture ont été réalisés sur lames après cytocentrifugation des cellules. Deux anticorps polyclonaux ont été utilisés : ler) l'anticorps C19, 2ème) l'anticorps de chèvre anti lapin FITC (fluorescéine) (TEBU). Des cellules HMCI (provenant d'un mastocytome humain) ont été utilisées comme témoin positif.

- Après cytocentrifugation les cellules sont fixées avec du méthanol pendant 5 mn.
- Elles sont ensuite séchées à l'air et rincées à 2 reprises en PBS+BSA 1% pendant 5 mn.
- Incubées ensuite avec du sérum de chèvre à 5% pendant 20 mn à température ambiante en chambre humide.
- 2 rinçages de 5 mn en PBS+BSA 1%.
- Incubées avec le 1er anticorps (C 19 au 1/50ème) 1h30mn à 37°C en chambre humide.
- 2 rinçages en PBS+BSA 1% (2 x 5 mn).
- Incubées avec le 2ème anticorps (chèvre anti-lapin FITC au 1/200ème) 1 h 30mn à 37°C en chambre humide.
- 2 rinçages de 5 mn en PBS.
- Les lames sont montées en mowiol.

La lecture est effectuée au microscope à fluorescence.

On observe un marquage positif membranaire sur la quasi-totalité des cellules ; ce marquage est identique à celui observé sur les cellules témoins HMCI, ce qui montre que les cellules analysées possèdent des récepteurs c-kit.

*Récepteur des IgE*

Des marquages directs à différents temps de la culture ont été réalisés en cytométrie de flux sur les mastocytes en culture avec un anticorps anti IgE de souris FITC. On a utilisé comme témoin positif des cellules RBL (lignée de basophiles de rat transformés en mastocytes).

- On utilise 1 ml de cellules en culture à environ $10^6$ cellules/ml pour chaque tube.
- Les cellules sont centrifugées pour éliminer le milieu de culture.
- Les culots sont repris dans 1 ml de PBS+BSA 1%, et incubés pendant 30 mn à +4°C.
- Les tubes sont centrifugés et les culots sont repris dans 100 $\mu$l d'anticorps anti IgE souris FITC dilué au 4/100eme, incubés 1h30 mn à +4°C.
- On effectue un rinçage dans 2 ml de PBS, puis les tubes sont centrifugés.
- Les culots sont recueillis dans 1 ml de PBS.

La lecture est faite en FACS.

A partir de J60 pour les cultures issues de la moelle osseuse, et de J67 pour les cultures issues du foie, 99% des cellules sont marquées ; le marquage est identique sur les cellules témoins RBL. Il apparaît donc que les cellules analysées possèdent le récepteur à l'IgE.

A J639, les profils de FACS montrent, pour les mastocytes du foie, deux populations cellulaires ; une population qui exprime faiblement les récepteurs aux IgE (17,5%) et une population qui exprime fortement les récepteurs aux IgE 82,5%.

*Analyse de l'ADN génomique des cellules*

20 microsatellites du panel diversifié du génome de porc ont été analysés à partir de l'ADN des cellules en culture (prélevé 489 jours après la mise en culture) et de celui du verrat et de la truie parents des 7 foetus sur lesquels ont été prélevés le foie et la moelle osseuse. Les marqueurs choisis sont très polymorphes et répartis sur la quasi-totalité des chromosomes.

Les résultats obtenus sont les suivants :

- La vérification mendélienne est satisfaisante, les cellules proviennent bien de foetus dont les parents étaient identifiés ;
- Pour 4 marqueurs principaux identifiés, IGFI, S0026, S0226 et SW240, les tailles observées correspondent aux allèles majoritaires ;
- Quel que soit le marqueur, les génotypes des différents échantillons d'ADN des cultures de mastocytes de foie foetal, et de moelle foetale sont identiques.

L'ADN génomique des cellules n'a pas été modifié pendant la culture (au moins pendant 489 jours, date de la préparation des échantillons d'ADN).

**Isolement de populations pures de mastocytes a partir de moelle ou de foie de foetus de porc de race Large White**

Les prélèvements ont été réalisés à partir de 5 foetus d'une truie Large White au 77$^{ème}$ jour de gestation; 7 foies et 8 fémurs ont été prélevés.

Les cellules ont été isolées et mises en culture, à partir du foie ou de la moelle, comme décrit ci-dessus pour les cellules foetales de race Meishan.

Des cultures pures de mastocytes de moelle osseuse ou de mastocytes de foie ont été obtenues après 112 jours de culture.

**EXEMPLE 2 : OBTENTION DE LIGNEES DE MASTOCYTES DE PORC TRANSFECTEES PAR UN ONCOGENE.**

**Immortalisation des cellules en culture**

Un prélèvement de $2.10^5$ mastocytes de foie foetal à 202 jours de culture est transfecté par lipofection en utilisant le vecteur pLM13, préalablement linéarisé par l'enzyme de restriction *Sca*1 associé à un liposome artificiel, FuGENE6 (BOEHRINGER-MANNHEIM). Le vecteur pLM13 comprend les séquences codant pour un mutant thermosensible de l'oncogène T (Tts) du virus simien vacuolisant SV40, et celles codant pour le gène néo (résistance à la généticine). Ces séquences sont sous le contrôle d'un promoteur de cytomégalovirus.

La présence de l'oncogène Tts dans l'ADN génomique des cellules transfectées a été observée par PCR. L'expression de Tts a été révélée par RT-PCR à partir d'une préparation d'ARN messagers des mêmes cellules.

**Croissance des mastocytes transfectés**

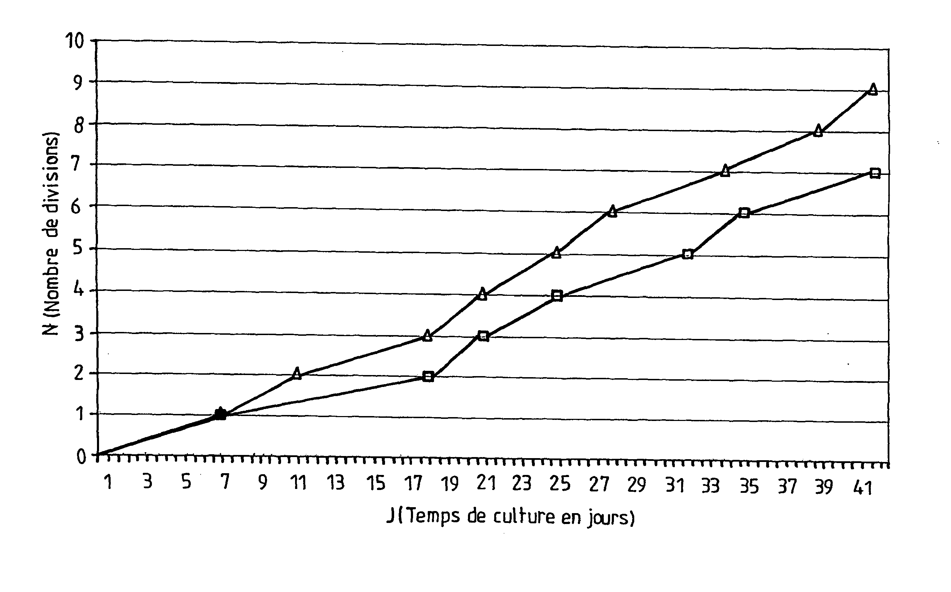

Une courbe de croissance a été réalisée sur les cellules non transfectées et sur les cellules transfectées de J470 à J511, soit pendant 41 jours de culture. A partir d'une concentration initiale de $5.10^5$ cellules/ml, les cellules ont été comptées tous les jours et rapportées à cette concentration initiale, pour évaluer le nombre de divisions. Les résultats sont illustrés par la Figure 1. En abscisse, le temps de culture (en jours) ; en ordonnée le nombre de divisions.

On observe une différence de croissance entre les mastocytes de foie non transfectés (□) et transfectés (Δ). Le temps de division pour les mastocytes non transfectés est en moyenne de 6 jours, alors qu'il est de 5 jours pour les mastocytes des foies transfectés (Figure 1).

**Caractérisation des lignées obtenues**

Les cultures de mastocytes transfectés présentent, au niveau de leur morphologie et de la présence d'héparine, les mêmes caractéristiques que les cultures de mastocytes non-transfectés.

Elles possèdent également les mêmes caractéristiques du récepteur c-kit. Toutefois, en ce qui concerne le récepteur des IgE, au lieu des deux populations cellulaires observées à J639 dans le cas des cultures de mastocytes de foie non transfectés, on observe une seule population.

**EXEMPLE 3 : PRODUCTION D'HISTAMINE PAR LES MASTOCYTES EN CULTURE.**

**Dosages d'histamine dans les mastocytes**

Des dosages d'histamine ont été réalisées sur les trois lignées cellulaires CNCM I-2735 (foie), CNCM I-2736 (foie transfecté par l'oncogène Tts de SV40) et CNCM I-2734 (moelle osseuse transfectée par l'oncogène Tts de SV40) à 976 jours de culture. Les cellules sont placées dans du milieu MEMα complet sans sérum.

1) Préparation des cellules :

50000 cellules de chaque lignée ont été activées pendant 30 minutes à 37°C en présence de 5% de $CO_2$, par 500 $\mu$M d'ionophore calcique A23187 et 5 $\mu$M de PMA (phorbol myristate acétate). Le même nombre de cellules de chaque lignée a été placé à 37°C pour servir de contrôle non-activé.

Les cellules sont ensuite centrifugées et les surnageants ainsi que les culots sont récupérés et congelés à -80°C jusqu'au moment du dosage de l'histamine.

2) Dosage de l'histamine :

Le dosage de l'histamine a été réalisé dans les surnageants et les culots selon la technique décrite pat LEBEL (Ann. Biochem. 133, 16-29, 1983).
Les résultats sont illustrés par le Tableau II ci-dessous.

TABLEAU II

| LIGNEE/TRAITEMENT | HISTAMINE DANS SURNAGEANT (ng/ml) | HISTAMINE DANS CULOT CELLULAIRE (ng/$10^6$ cellules) | LIBERATION D'HISTAMINE (%) |
|---|---|---|---|
| I-2735 non activée | 14 | 1583 | 48 |
| I-2735 activée | 449 | 466 | |
| I-2734 non activée | 16 | 1460 | 28 |
| I-2734 activée | 250 | 600 | |
| I-2736 non activée | 23 | 4380 | 25 |
| I-2736 activée | 593 | 1760 | |

Toutes les cellules contiennent de l'histamine. La concentration d'histamine dans les surnageants est plus importante dans les cellules activées que dans les cellules non activées.
Le pourcentage de libération d'histamine par les cellules activées est calculé en utilisant la formule suivante :

$$\% \text{ de libération} = \frac{S - S \text{ Contrôle x } 100}{(S+P) - S \text{ Contrôle}}$$

dans laquelle :

S= Histamine dans le surnageant
C= histamine dans le culot.

**Revendications**

1.  Procédé d'obtention d'une culture de mastocytes porcins, **caractérisé en ce qu'**il comprend la mise en culture de cellules souches obtenues à partir du foie ou de la moelle osseuse d'un foetus de porc, dans un milieu comprenant au moins 0,2 ng/ml d'IL-3 porcin et au moins 8 ng/ml de SCF porcin.

2.  Procédé selon la revendication 1, **caractérisé en ce que** lesdites cellules sont maintenues en culture dans ledit milieu pendant au moins 60 à 70 jours.

3.  Procédé selon une quelconque des revendications 1 ou 2, **caractérisé en ce qu'**il comprend en outre la transformation de cellules de ladite culture par un oncogène immortalisant.

4.  Culture de mastocytes porcins susceptible d'être obtenue par un procédé selon une quelconque des revendications 1 à 3.

5.  Culture de mastocytes porcins selon la revendication 4, choisie parmi :

    - la lignée déposée auprès de la CNCM le 17 octobre 2001, sous le numéro I-2735 ;
    - la lignée déposée auprès de la CNCM le 17 octobre 2001, sous le numéro I-2736 ;
    - la lignée déposée auprès de la CNCM le 17 octobre 2001, sous le numéro I-2734.

6.  Utilisation d'une culture de mastocytes porcins selon une quelconque des revendications 4 ou 5 en tant que modèle d'étude de l'activation mastocytaire.

7.  Utilisation d'une culture de mastocytes porcins selon une quelconque des revendications 4 ou 5, pour le criblage *in vitro* d'antigènes capables d'induire l'activation des mastocytes.

**Claims**

1. Method for obtaining a pig mast cell culture, **characterized in that** it comprises culturing stem cells obtained from the liver or bone marrow of a pig foetus, in a medium comprising at least 0.2 ng/ml of porcine IL-3 and at least 8 ng/ml of porcine SCF.

2. Method according to claim 1, **characterized in that** said cells are maintained in culture in said medium for at least 60 to 70 days.

3. Method according to any one of claims 1 or 2, **characterized in that** it also comprises transforming cells of said culture with an immortalizing oncogene.

4. Pig mast cell culture which may be obtained by a method according to any one of claims 1 to 3.

5. Pig mast cell culture according to claim 4, selected from among:

   - the line deposited at the CNCM on October 17, 2001, under the number 1-2735;
   - the line deposited at the CNCM on October 17, 2001, under the number 1-2736;
   - the line deposited at the CNCM on October 17, 2001, under the number 1-2734.

6. Use of a pig mast cell culture according to any one of claims 4 or 5 as a model for studying mast cell activation.

7. Use of a pig mast cell culture according to any one of claims 4 or 5, for *in vitro* screening of antigens capable of inducing mast cell activation.

**Patentansprüche**

1. Verfahren zum Erhalt einer Schweine-Mastzellen-Kultur, **dadurch gekennzeichnet, dass** sie das Kultivieren von Stammzellen umfasst, die aus Gans oder dem Knochenmark eines Schweinefötus erhalten wurden, in einem Medium, das wenigstens 0,2 ng/ml Schweine-IL-3 umfasst und wenigstens 8 ng/ml Schweine-Stammzellfaktor (SCF).

2. Verfahren nach Anspruch 1, **dadurch gekennzeichnet, dass** die Zellen für wenigstens 60 bis 70 Tage in dem Medium kultiviert werden.

3. Verfahren nach einem der Ansprüche 1 oder 2, **dadurch gekennzeichnet, dass** es unter anderem die Transformation der Zellen der Kultur mit einem immortalisierenden Onkogen umfasst.

4. Schweine-Mastzellen-Kultur, erhältlich nach einem Verfahren nach einem der Ansprüche 1 bis 3.

5. Schweine-Mastzellen-Kultur nach Anspruch 4, ausgewählt aus:

   - der Zelllinie, die bei der CNCM am 17. Oktober 2001 unter der Nummer I-2735 hinterlegt wurde
   - der Zelllinie, die bei der CNCM am 17. Oktober 2001 unter der Nummer I-2736 hinterlegt wurde
   - der Zelllinie, die bei der CNCM am 17. Oktober 2001 unter der Nummer I-2734 hinterlegt wurde.

6. Verwendung einer Schweine-Mastzellen-Kultur nach einem der Ansprüche 4 oder 5 als Unteruchungsmodell der Mastzellen-Aktivierung.

7. Verwendung einer Schweine-Mastzellen-Kultur nach einem der Ansprüche 4 oder 5, für die *in vitro* Selektion von Antigenen, die geeignet sind die Aktivierung von Mastzellen zu induzieren.